# EUROPEAN PATENT SPECIFICATION

(11) **EP 2 736 553 B1**
(45) Date of publication and mention of the grant of the patent: **30.12.2015**
(21) Application number: 11743726.9
(22) Date of filing: 29.07.2011
(51) Int. Cl.: A61M 1/16

(54) **SODIUM MANAGEMENT FOR DIALYSIS SYSTEMS**
NATRIUMVERWALTUNG FÜR DIALYSESYSTEME
GESTION DU SODIUM POUR SYSTÈMES DE DIALYSE

(43) Date of publication of application: 04.06.2014
(73) Proprietor: Baxter International Inc., Deerfield, IL 60015 (US); Baxter Healthcare S.A., 8152 Glattpark (Opfikon) (CH)
(72) Inventor: DING, Yuanpang Samuel, Libertyville, Illinois 60048 (US); LIN, Rongsheng, Buffalo Grove, Illinois 60089 (US); LO, Ying-Cheng, Green Oaks, Illinois 60048 (US)
(74) Representative: Potter Clarkson LLP
(86) International application number: PCT/US2011/045887
(87) International publication number: WO 2013/019179

(56) References cited:
- FR-A1- 2 570 948
- US-A- 4 542 015
- US-A1- 2005 274 658
- US-A1- 2007 213 665
- US-A1- 2009 101 552

## Description

### BACKGROUND

The present disclosure relates generally to dialysis therapies. More specifically, the present disclosure relates to sodium management for dialysis systems such as wearable kidneys.

Hemodialysis and peritoneal dialysis are two types of dialysis therapies used commonly to treat loss of kidney function. A hemodialysis treatment filters the patient's blood to remove waste, toxins and excess water from the patient. The patient is connected to a hemodialysis machine and the patient's blood is pumped through the machine. Catheters are inserted into the patient's veins and arteries so that blood can flow to and from the hemodialysis machine. The blood passes through a dialyzer of the machine, which removes waste, toxins and excess water from the blood into a fluid called dialysate that also passes through the dialyzer. The cleaned blood is returned to the patient. A large amount of dialysate, for example about 120 liters, is consumed to dialyze the blood during a single hemodialysis therapy. Hemodialysis treatment typically lasts several hours and is generally performed in a treatment center about three or four times per week.

Peritoneal dialysis uses a dialysis solution, also called dialysate, which is infused into a patient's peritoneal cavity via a catheter. The dialysate contacts the peritoneal membrane of the peritoneal cavity. Over a period of one or more hours, waste, toxins and excess water pass from the patient's bloodstream, through the peritoneal membrane and into the dialysate due to diffusion and osmosis, i.e., an osmotic gradient occurs across the membrane. The spent dialysate is then drained from the patient, removing waste, toxins and excess water from the patient. This cycle is repeated several times daily.

There are various types of peritoneal dialysis therapies, including continuous ambulatory peritoneal dialysis ("CAPD"), automated peritoneal dialysis ("APD"), tidal flow APD and continuous flow peritoneal dialysis ("CFPD"). CAPD is a manual dialysis treatment. The patient manually connects an implanted catheter to a drain, allowing spent dialysate fluid to drain from the peritoneal cavity. The patient then connects the catheter to a bag of fresh dialysate, infusing fresh dialysate through the catheter and into the patient. The patient disconnects the catheter from the fresh dialysate bag and allows the dialysate to dwell within the peritoneal cavity, wherein the transfer of waste, toxins and excess water takes place. After a dwell period of several hours, the patient repeats the manual dialysis procedure, for example, four times per day with each procedure taking about an hour. Manual peritoneal dialysis requires a significant amount of time and effort from the patient, leaving ample room for improvement.

APD is similar to CAPD in that the dialysis treatment includes drain, fill, and dwell cycles. APD machines, however, perform the cycles automatically, typically while the patient sleeps. APD machines free patients from having to manually perform the treatment cycles and from having to transport supplies during the day. An APD machine connects fluidly to the patient's implanted catheter, to a source of fresh dialysate, and to a fluid drain. The dialysate source can be one or several sterile dialysate solution bags. The APD machine pumps fresh dialysate from the dialysate source, through the catheter, into the patient's peritoneal cavity, and allows the dialysate to dwell within the cavity so that the transfer of waste, toxins and excess water can take place. After a specified dwell time, the APD machine pumps spent dialysate from the peritoneal cavity, though the catheter, to the drain. As with the manual process, several drain, fill and dwell cycles occur during APD. A "last fill" may occur at the end of a CAPD or APD cycle, whereby the dialysate remains in the patient's peritoneal cavity of the until the next treatment.

Both CAPD and APD are batch type systems in which spent dialysis fluid is drained from the patient and discarded. One alternative to batch systems is a tidal flow system. This is a modified batch system in which a portion of the fluid is removed and replaced after smaller increments of time instead of removing all of the fluid from the patient after a longer period of time.

Continuous flow, or CFPD, dialysis systems clean or regenerate spent dialysate instead of discarding it. These systems pump fluid into and out of the patient, through a loop. Dialysate flows into the peritoneal cavity through one catheter lumen and out another catheter lumen. The fluid exiting the patient passes through a reconstitution device that removes waste from the dialysate, e.g., via a urea removal column that employs urease to enzymatically convert urea into ammonia (e.g., ammonium cation). The ammonia is then removed from the dialysate by adsorption before reintroduction of the dialysate into the peritoneal cavity. Additional sensors are employed to monitor the removal of ammonia. CFPD systems are typically more complicated than batch systems.

In both hemodialysis and peritoneal dialysis, "sorbent" technology can be used to remove uremic toxins from used dialysate and replenish depleted therapeutic agents (such as ions and/or glucose) in the treated fluid, so that the treated fluid may be reused to continue the dialysis of the patient. One commonly used sorbent is made from zirconium phosphate, which is used to remove ammonia generated by the hydrolysis of urea. Typically, a large quantity of sorbent is necessary to remove the ammonia generated during dialysis treatments.

The main advantage of the sorbent based approach is that lower volumes of dialysis fluid or dialysate are required to achieve high volume dialysis treatments. The main disadvantages of sorbent systems are the high cost of the sorbent, the amount of space required to house the sorbent, and concerns regarding the purity of the recycled solution, as many ions remain in the fluid after treatment and it is technically challenging to verify purity. In particular, the level of sodium in a sorbent treated dialysis solution can become a concern. For example, sodium level in the dialysate should not be higher than 140 millimoles/L ("mM") during hemodialysis to allow sodium removal from the patient.

US 2005/0274658 discloses a dialysis system comprising a first housing containing a material that is capable of decomposing urea from the dialysate flowing through the first housing to release sodium ions and second housing containing a material that binds sodium ions from the dialysate flowing through the second housing. The first and second housings are in fluid communication with one another so that dialysate flows from a dialyzer through the first housing, the second housing and then back to the dialyzer. A suitable material in the second housing is a mixed bed ion exchange resin containing a cation exchange resin and an anion exchange resin.

### SUMMARY

The present disclosure relates to improved dialysis cartridges for sodium management as well as methods for providing dialysis to a patient. In one embodiment, the present disclosure provides an apparatus for dialysis treatment comprising first and second fluid flow pathways in a parallel arrangement. The first fluid flow pathway contains a first cation exchange resin, wherein greater than 90% of exchange sites of the first cation exchange resin are populated with hydrogen ions, and the second fluid flow pathway contains a second cation exchange resin, wherein greater than 90% of exchange sites of the second cation exchange resin are populated with sodium ions. A total ion exchange capacity ratio of the first cation exchange resin compared to the second cation exchange resin ranges from about 1:1 to about 1:5. The apparatus can further include, in association with the first and second fluid flow pathways, at least one layer of material such as urease, zirconium oxide, carbon or a combination thereof.

In another embodiment, the apparatus further includes a third fluid flow pathway in substantially parallel flow arrangement with said first and second fluid flow pathways, said third pathway comprising an anion exchange resin. From about 20% to about 80% exchange sites of the anion exchange resin are populated with carbonate or bicarbonate ions. The total ion exchange capacity ratio of the first cation exchange resin compared to the anion exchange resin can range from about 1:0 to about 1:2. In association with the first, second and third fluid flow pathways, the apparatus can include at least one layer selected from the group consisting of a urease layer, a zirconium oxide layer, a carbon layer and combinations thereof.

Also described is a method of managing sodium during a dialysis therapy. The method includes circulating a spent dialysis fluid in a fluid circuit that includes a dialysis cartridge including a first fluid flow pathway having a first cation exchange resin, wherein greater than 90% exchange sites are populated with hydrogen ions, and a second fluid flow pathway having a second cation exchange resin, wherein greater than 90% of exchange sites are populated with sodium ions. The second fluid flow pathway is in a parallel flow arrangement with the first fluid flow pathway. The method further includes removing ions in the dialysis fluid with the cartridge to produce a regenerated dialysis fluid, and recirculating the regenerated dialysis fluid back to a patient.

One method includes supplementing the regenerated dialysis fluid with a dialysis component such as calcium, magnesium, potassium, acetate, bicarbonate or a combination thereof.

An advantage of the present disclosure is to provide an improved dialysis fluid cleaning cartridge for providing sodium management.

Another advantage of the present disclosure is to provide an improved method for managing sodium levels in portable dialysis cartridges including cartridges employing sorbent or spent fluid cleaning technology.

Still another advantage of the present disclosure is to provide an improved method for providing dialysis.

Yet another advantage of the present disclosure is to provide an improved dialysis fluid cleaning cartridge that can be used in a single loop or a multiple loop dialysis system.

An alternative advantage of the present disclosure is to provide an improved resin for a sorbent cartridge to be used in a dialysis system.

Additional features and advantages are described herein, and will be apparent from, the following Detailed Description and the figures.

### BRIEF DESCRIPTION OF THE FIGURES

FIG. 1 illustrates a dialysis cartridge for providing sodium management in an embodiment of the present disclosure.
FIG. 2 illustrates a dialysis cartridge for providing sodium management in a second embodiment of the present disclosure.
FIG. 3 illustrates a dialysis cartridge for providing sodium management in a third embodiment of the present disclosure.
FIGS. 4A to 4D are schematic illustrations of the dialysis cartridges used in various dialysis treatment technologies.
FIG. 5 shows a graph of the sodium and ammonium elution curves of a zirconium phosphate column in acidic form.
FIG. 6 shows a graph of the sodium and ammonium elution curves of a zirconium phosphate column in sodium form.
FIG. 7 shows a graph of the sodium and ammonium elution curves of the combined zirconium phosphate columns.
FIG. 8 shows a graph of the bicarbonate and pH elution curves of the combined zirconium phosphate columns.

### DETAILED DESCRIPTION

### Systems and Methods

The present disclosure relates to improved dialysis systems that in general reuse and/or replenish spent dialysis fluid for providing sodium management as well as methods for providing dialysis to a patient. The dialysis systems and methods can be used and implemented in various hemodialysis and peritoneal dialysis technologies such as, for example, those described in U.S. Patent Nos. 5,244,568; 5,350,357; 5,662,806; 6,592,542; and 7,318,892. The hemodialysis and peritoneal dialysis technologies can be designed and configured for medical centers and be implemented with on-site or at-home dialysis treatments. The dialysis systems and methods can further be used in portable dialysis systems such as, for example, wearable artificial kidneys in which a patient may move freely during dialysis. The portable dialysis devices can also encompass transportable dialysis devices (e.g., dialysis devices that are sized to be transported by a user), which are not needed to be fixed in one place such as a hospital. Non-limiting examples of portable dialysis systems are described in U.S. Patent Nos. 5,873,853; 5,984,891; and 6,196,992 and U.S. Patent Publication Nos. 2007/0213665 and 2008/0051696.

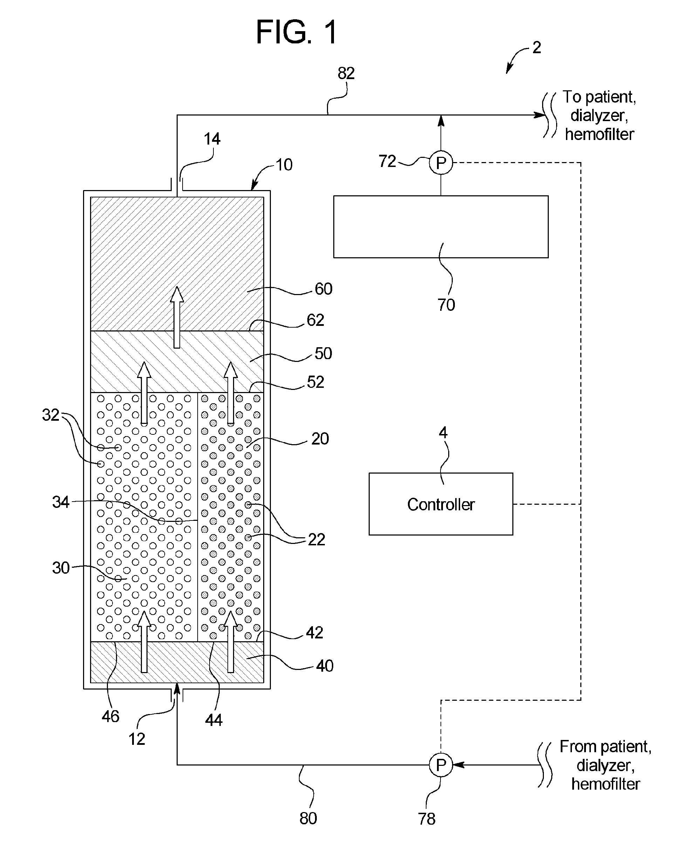

Referring now to the drawings and in particular to FIG. 1, one embodiment of a dialysis system 2 of the present disclosure is illustrated. Dialysis system 2 includes a cartridge 10 having an inlet 12 and an outlet 14. Cartridge 10 includes a first column 20 having a first cation exchange resin 22 (solid circles) in which greater than 90% exchange sites are populated with hydrogen ions (e.g., in acidic form). Cartridge 10 further includes a second column 30 having a second cation exchange resin 32 (empty circles) in which greater than 90% of exchange sites are populated with sodium ions (e.g., in neutral form). Second column 30 can be separated from first column 20 using any suitable barrier 34 such, for example, a plastic impermeable barrier. Second column 30 can be parallel with first column 20. The total ion exchange capacity ratio of first cation exchange resin 22 contained in first column 20 compared to the second cation exchange resin 32 contained in second column 30 can range from about 1:1 to about 1:5.

As used herein, the term "parallel," can mean parallel, approximately parallel, substantially parallel, or side-by-side. As used herein, the term "total ion exchange capacity" can mean the theoretical number of exchangeable ions per unit volume of an ion exchange resin. For example, the total ion exchange capacity (e.g., in units of milli-equivalents ("mEq")) of a column having an ion exchange resin is the specific ion exchange capacity (e.g., in mEq/gram) of the resin multiplied by the amount of resin (e.g., in grams) in the column.

In an embodiment, first cation exchange resin 22 and second cation exchange resin 32 are zirconium phosphate resins. It has been surprisingly found that the mostly or fully sodium neutralized form of zirconium phosphate releases an almost constant level of sodium ions from the cation exchange resin. A mostly or fully acidic form of zirconium phosphate is also provided and removes all or a certain constant level of sodium from the dialysate. In other words, the sodium concentration in the effluent fluid from first column 20 is found to have an almost constant but reduced level of sodium relative to the influent fluid, and the sodium concentration of the effluent fluid from second column 30 is found to have an almost constant but increased level of sodium relative to the influent fluid.

By combining first column 20 and second column 30 in parallel, an optimal dialysate volume flow rate ratio can be obtained in these two columns that provides a targeted and constant level of sodium concentration to be maintained in the effluent dialysate leaving cartridge 10. The volume flow rate (e.g., in units of volume/time such as milliliters per minute ("ml/min") or ml per second ("ml/sec")) of the fluid into first column 20 relative to the volume flow rate into second column 30 can be adjusted to provide a targeted effluent fluid that has an almost constant and close to the desirable target sodium concentration. For example, a targeted sodium concentration in the regenerated dialysate during hemodialysis is about 140 mM. An exemplary targeted sodium concentration in the regenerated dialysate during peritoneal dialysis is about 132 mM.

The volume flow rate of the fluid into first column 20 relative to the volume flow rate into second column 30 can be adjusted by setting the inlet surface areas 44 and 46 relative to each other to form desirable relative volume flow rates in columns 20 and 30, respectively. Assuming that the density of exchange resin 22 and exchange resin 32 are roughly the same and that urease layer 40 is uniformly distributed upstream of columns 20 and 30, the velocity of fluid flowing through columns 20 and 30 should be the same; that is, the pressure inside cartridge 10 should be uniform. The larger cross-sectional surface area of column 30 relative to column 20, in combination with the uniform flow velocity, will create an overall increase in the volume flow rate in larger column 30 than in smaller column 20. It should be appreciated, however, that on a resin particle by resin particle basis, the flow velocity that a particle of resin in each column 20 and 30 sees is roughly the same due to the uniform velocity. Another way of saying this is that a cubic centimeter of resin 22 and a cubic centimeter of resin 32 will see the same flow velocity of spent dialysis fluid. In this embodiment then, effective effluent dialysate cleansing is achieved by providing more volume or mass of one of resin 22 and resin 32 and a constant velocity across a given cross-section of cartridge 10.

In another embodiment, the velocity of effluent flowing through a cubic centimeter of resin 22 is varied relative to a velocity of effluent flow rate through a cubic centimeter of resin 32, thus varying a volume flow rate of the two resins even if the overall mass or volume of resin 22 and resin 32 in cartridge 10 are equal. The flow velocity can be varied in different ways. In one way, the flow velocity is varied by selecting the respective packing densities of the materials of first column 20 and second column 30 shown in FIG. 1, so that it is relatively easier to flow through one column as opposed to the other column. This permits different volume flow rates through each column 20 and 30 even if the columns have the same volume.

Another way of varying velocity is to place a flow restrictor (e.g., a narrow tubing section) at the inlet or outlet of one or both of columns 20 and 30 to control the relative volume flow rate between the columns.

A further way of varying relative velocity is to provide inlet valves that control fluid flow into first column 20 thus permitting adjustment of the volume flow rate of the fluid into first column 20 relative to the volume flow rate into second column 30. For example, barrier 34 could be extended all the way to the wall of inlet 12. Here, urease layer 40 is still provided, but split between the two columns 20 and 30. Inlet 12 is then split into two inlets, one for each column 20 and 30. Each of the split inlets is provided with a dedicated inlet valve, only one of which is open at any given time. The inlet valves can be toggled on and off to provide different percentage open times (e.g., sixty percent open for column 30 versus forty percent for column 20, resulting in roughly a 3:2 flow rate through column 30 versus column 20 assuming roughly the same inlet pressure to both valves). Alternatively, the two dedicated inlet valves may be replaced with a switching valve that is configured to direct flow to columns 20 and 30 on a time-proportional basis. In another embodiment, the inlet valves are varying orifice valves that are set individually to create the desired relative volume flow rates in column 20 versus column 30. In either valve case, it should be appreciated that columns 20 and 30 need not be sized differently to achieve different flow rates.

In another embodiment, first cation exchange resin 22 has greater than 95% of exchange sites populated with hydrogen ions, and second cation exchange resin 32 has greater than 95% exchange sites populated with sodium ions. In an alternative embodiment, first cation exchange resin 22 has greater than 99% of exchange sites populated with hydrogen ions, and second cation exchange resin 32 has greater than 99% exchange sites populated with sodium ions.

As further shown by FIG. 1, dialysis cartridge 10 can include one or more of a urease layer 40, a zirconium oxide layer 50 and/or a carbon layer 60 in any suitable form (e.g., beads, particles, etc.). Urease layer 40 in the illustrated embodiment is provided closest to inlet 12. Urease layer 40 can be followed by first column 20 and second column 30 as shown. Zirconium oxide layer 50 can follow first column 20 and second column 30. Carbon layer 60 can be closest to outlet 14.

Although one specific order of different layers in dialysis cartridge 10 is shown in FIG. 1, it should be appreciated that the urease layer 40, zirconium oxide layer 50 and/or carbon layer 60 can be arranged in other suitable orders to optimize the performance of dialysis system 2 according to the objectives of the user. In addition, permeable layers 46, 52 and 62 can be used to separate any of the aforementioned layers in cartridge 10. Permeable layers 46, 52 and 62 can be made from any suitable fluid permeable material such as, for example, filter paper or a hydrophilic material. In addition, if desired, any of the urease layer 40, zirconium oxide layer 50, or carbon layer 60 may be provided in a separate cartridge or vessel to permit replacement of one or more layers independently.

During use of dialysis system 2, a pump 78, such as a membrane or peristaltic pump, pumps spent or effluent dialysis fluid through line 80 and into inlet 12 of dialysis cartridge 10. The spent dialysis fluid passes through the different layers of dialysis cartridge 10, so that each layer removes one or more effluent compounds from the spent dialysis fluid stream introduced through line 80. A regenerated dialysis stream exits outlet 14 of dialysis cartridge 10 through regenerated dialysate line 82. Any one or more dialysis supplement components, such as calcium, magnesium, potassium, bicarbonate, acetate and/or other suitable electrolyte, can be added from one or more sources 70 via one or more substitution pumps 72, which can be of any type described for pump 78, to regenerated dialysis line 82 after regenerated dialysis fluid exits dialysis system 2.

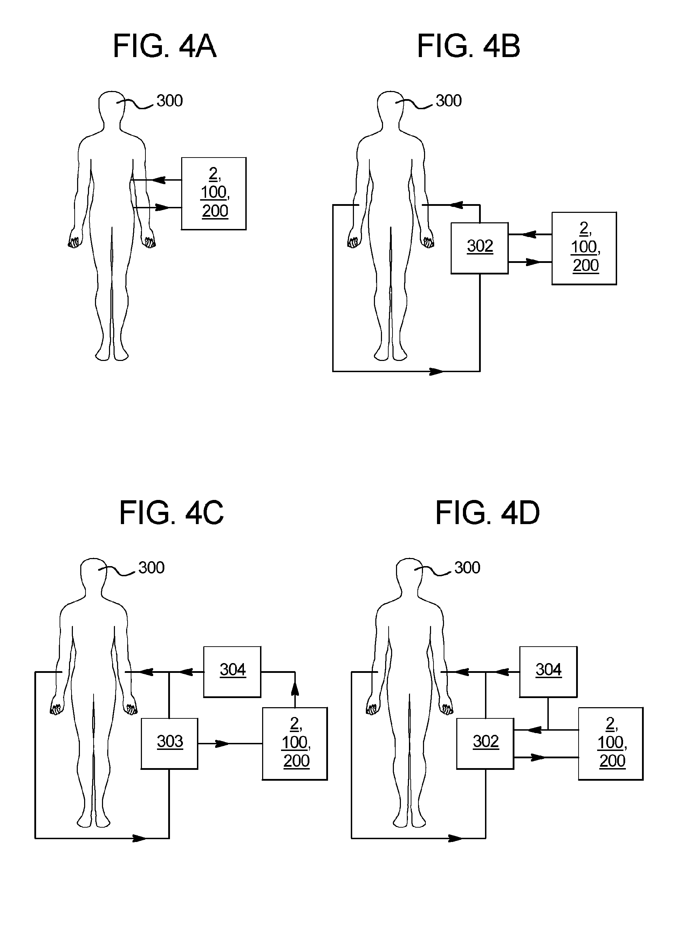

Controller 4 controls pumps 72 and 78 as needed to reach the desired dose of additives and to achieve the desired flow through cartridge 10, respectively. Controller 4, like all controllers discussed herein, can include one or more processors and memories and can control other features of dialysis system 2 of FIGS. 4A to 4D or can be a delegate controller dedicated to a supervisory controller or control unit of dialysis system 2. Other features of dialysis system 2 (as well as any of the systems herein) can include the control of components for dialysate mixing, dialysate heating, dialysate flow and volume control (e.g., to and from a patient dialyzer or hemofilter) and ultrafiltration control.

The flow regime of system 2 of FIG. 1 has been simplified to highlight certain features. It should be appreciated that dialysis lines 80 and 82 can be fitted with one or more control or monitoring components including one or more of a pressure gauge, flow meter, conductivity probe (e.g., temperature compensated) and/or valve.

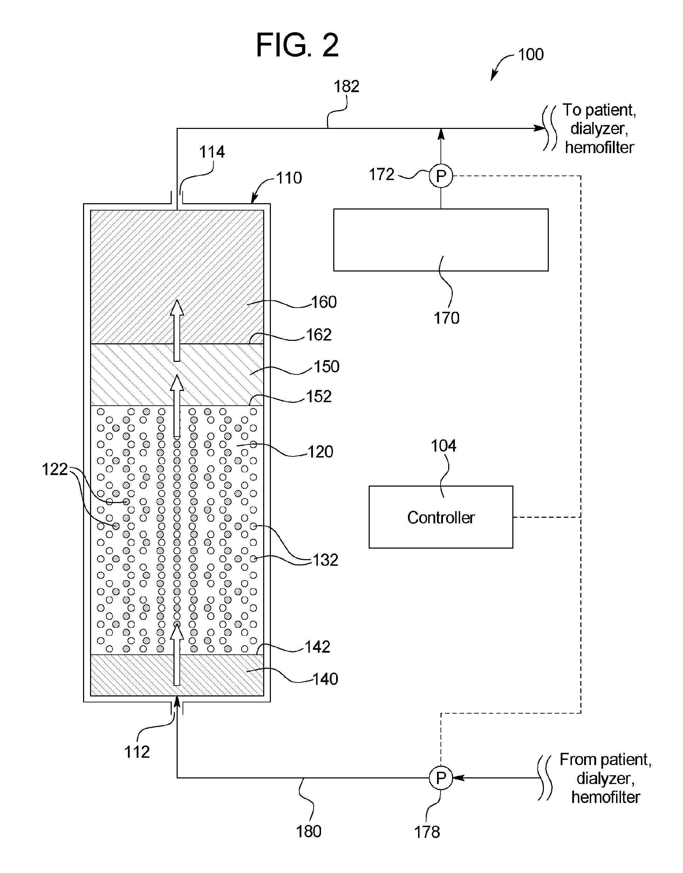

Referring to FIG. 2, another embodiment of the present disclosure is illustrated by dialysis system 100. Dialysis system 100 includes a cartridge 110 having an inlet 112 and an outlet 114. Cartridge 110 includes a column 120 containing a mixture of a first cation exchange resin 122 (solid circles) in which greater than 90% exchange sites are populated with hydrogen ions and a second cation exchange resin 132 (empty circles) in which greater than 90% of exchange sites are populated with sodium ions. The total ion exchange capacity ratio of first cation exchange resin 122 compared to second cation exchange resin 132 can range from about 1:1 to about 1:5.

In another embodiment, first cation exchange resin 122 has greater than 95% of exchange sites populated with hydrogen ions, and second cation exchange resin 132 has greater than 95% exchange sites populated with sodium ions. In another alternative embodiment, first cation exchange resin 122 has greater than 99% of exchange sites populated with hydrogen ions, and the second cation exchange resin 132 has greater than 99% exchange sites populated with sodium ions.

As further shown by FIG. 2, system 100 can include one or more of a urease layer 140, a zirconium oxide layer 150 and/or a carbon layer 160 in any suitable form. In the illustrated embodiment, urease layer 140 is closest to inlet 112. Urease layer can be followed by column 120. Zirconium oxide layer 150 can follow column 120. Carbon layer 160 can be closest to outlet 114.

Although one specific order of different layers in dialysis cartridge 100 is shown in FIG. 2, it should be appreciated that the urease layer 140, zirconium oxide layer 150 and/or carbon layer 160 can be arranged in other suitable orders to optimize the performance of system 100 according to the objectives of the user. In addition, permeable layers 142, 152 and 162 can be used to separate any of the aforementioned layers in cartridge 110. Permeable layers 142, 152 and 162 can be made from any suitable fluid permeable material such as, for example, filter paper or a hydrophilic material. In addition, if desired, any of the urease layer 140, zirconium oxide layer 150, or carbon layer 160 may be provided in a separate cartridge or vessel to permit replacement of one or more layers independently.

During use of dialysis system 100, a pump 178, such as a membrane or peristaltic pump, pumps spent or effluent dialysis fluid through line 180 so as to enter inlet 112 of dialysis cartridge 110. The spent dialysis fluid passes through the different layers of dialysis cartridge 110, so that each layer removes one or more effluent compounds from the spent dialysis fluid stream introduced through line 180. A regenerated dialysis stream exits outlet 114 of dialysis cartridge 110 through regenerated dialysate line 182. One or more dialysis supplements such as calcium, magnesium, potassium, bicarbonate, acetate and/or other suitable electrolytes can be added from one or more sources 170 via one or more substitution pumps 172, which can be any type described for pump 178, to regenerated dialysate line 182 after regenerated dialysis fluid exits dialysis system 100.

Controller 104 controls pumps 172 and 178 as needed to reach the desired dose of additives and to achieve the desired flow through cartridge 110, respectively. Controller 104 can include one or more processors and memories and, where dialysis system 100 is used, can control the other features of dialysis system 100 of FIGS. 4A to 4D. Controller 104 and system 100 can include any of the alternatives discussed above for controller 4 and dialysis system 2, respectively. For example, an on/off or variable restriction or orifice valve, flow regulator or flowmeter, providing feedback to controller 4, can be provided in line 180 to control the flow rate through cartridge 110 as desired.

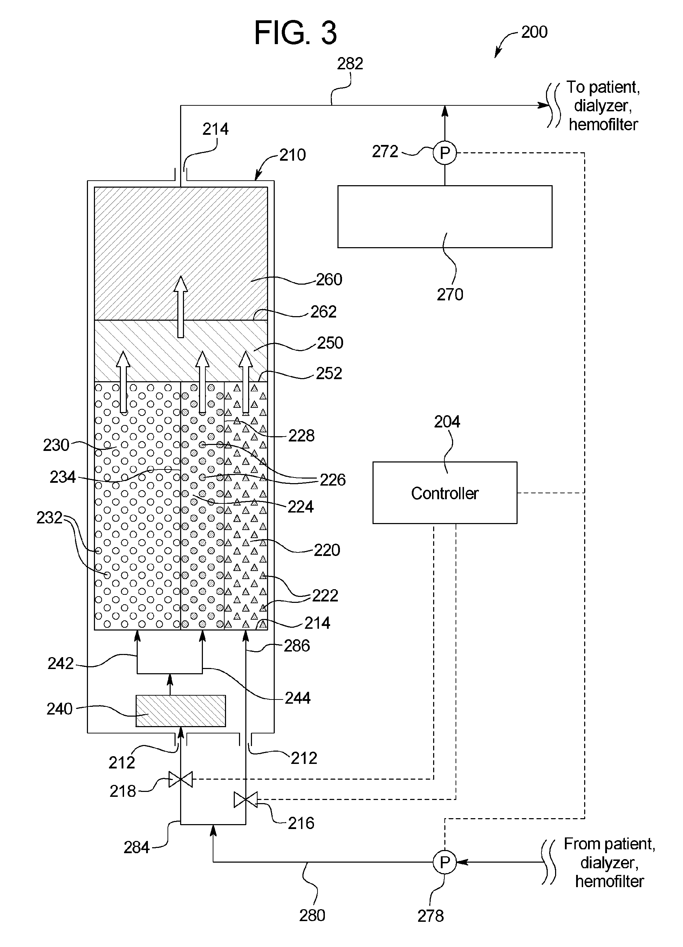

Referring to FIG. 3, a further alternative embodiment of the present disclosure is illustrated by dialysis system 200. Dialysis system 200 includes a cartridge 210 having a plurality of fluid inlets 212 and a fluid outlet 214. Cartridge 210 includes a first column 220, a second column 224 and a third column 230. First column 220, second column 224 and third column 230 can be separated by any suitable barriers 228 and 234, respectively, such as, for example, a plastic wall.

First column 220 is filled with an anion exchange resin 222 (triangles) in which from about 20% to about 80% of exchange sites are populated with carbonate or bicarbonate ions and from about 20% to about 80% of exchange sites are populated with hydroxide ions. Second column 224 is filled with a first cation exchange resin 226 (solid circles) in which greater than 90% exchange sites are populated with hydrogen ions. Third column 230 is filled with a second cation exchange resin 232 (empty circles) in which greater than 90% of exchange sites are populated with sodium ions. First column 220, second column 224 and third column 230 can be of approximately a same length and be approximately parallel with each other.

The acidic form of the cation exchange resin will release hydrogen (hydronium) ions and can absorb most metal cations and the ammonium ions. The carbonate or bicarbonate ions form of the anion exchange resin will release carbonate or bicarbonate ions anions and can absorb chloride, nitrate and sulfate anions, but will not significantly remove bicarbonate or acetate anions.

In an embodiment, the total ion exchange capacity ratio of first cation exchange resin 226 contained in second column 224 compared to the second cation exchange resin 232 contained in third column 230 ranges from about 1:1 to about 1:5. By adjusting the flow rate of the fluid into third column 230 relative to the flow rate of fluid into second column 224, the combined effluent fluid can have an almost constant and close to the desirable target sodium concentration. The flow rate into second column 224 relative to the flow rate of fluid into third column 230 can be controlled on a fixed velocity or fixed volume (varying velocity) basis as described above for columns 20 and 30 of FIG. 1. In the fixed velocity case, the cross-sectional area of column 224 is set relative to the cross-sectional area of column 230 so as to create a desired overall volume flow rate differential through the entire columns 224 and 230.

If it is desired to vary flow velocity through a fixed volume (e.g., one cm³) of resin 232 versus a fixed volume (e.g., one cm³) of resin 226, then one or more of the structures and methods described above for FIG. 1 can be used. For example, the restriction of inlet line 242 can be varied relative to the restriction of inlet line 244. The velocity of fluid entering each column 224 and 230 multiplied by the cross-sectional area of each column sets the flow rate through the column. Again, the cross-sectional areas of columns 224 and 230 can be the same. The varied velocities from varying restrictions of lines 242 and 244 will cause the volume flow rate to then vary in each column 224 and 230.

Alternatively, barriers 228 and 234 are extended all the way to the inlet wall for inlet 212. A separate inlet 212 is provided for each column 224 and 230. Each column 224 and 230 is separately valved. Fluid velocity into each column 224 and 230 is controlled individually by sequencing each valve (if on/off valves are provided) at a desired frequency or setting the variable orifices (if the valves are variable orifice valves) at different desired settings.

In another embodiment, the total ion exchange capacity ratio of first cation exchange resin 226 contained in second column 224 compared to the anion exchange resin 222 contained in first column 220 ranges from about 1:0 to about 1:2. If the solution pH of the combined effluent fluids from second column 224 and third column 230 is found to be acidic, the flow rates of the fluid into first column 220 can be adjusted relative to the flow rate into second column 224 and third column 230 (e.g., via valve 216) to adjust the combined effluent fluid pH to a desired range (e.g., around 7). Valve 216 like the other valves described herein can be an on/off valve that is sequenced to achieve a desired per unit volume flow rate within column 220 or be a variable orifice valve that is opened or closed an amount that achieves a desired per unit volume flow rate. Valves 216 and 218, like any other valves discussed herein, can be electronically controlled with an associated controller, e.g., controller 204 of FIG. 3.

In an embodiment, first cation exchange resin 226 has greater than 95% of exchange sites populated with hydrogen ions, second cation exchange resin 232 has greater than 95% exchange sites populated with sodium ions, and anion exchange resin 222 has greater than 95% exchange sites populated with carbonate or bicarbonate ions. In another embodiment, first cation exchange resin 222 has greater than 99% of exchange sites populated with hydrogen ions, second cation exchange resin 232 has greater than 99% exchange sites populated with sodium ions, and anion exchange resin 222 has from about 40% to about 60% exchange sites populated with carbonate or bicarbonate ions and about 40% to about 60% exchange sites populated with hydroxide ions.

As further shown by FIG. 3, dialysis cartridge 200 can include one or more of a urease layer 240, a zirconium oxide layer 250 and/or a carbon layer 260 in any suitable form and combination. In the illustrated embodiment, urease layer 240 is positioned closest to the inlets 212. In an embodiment, urease layer 240 is followed by third column 230 and second column 224, which contain cation exchange resins that can remove the ammonium ions generated by urease layer 240. Zirconium oxide layer 250 can follow first column 220, second column 224 and third column 230. Carbon layer 260 is positioned closest to outlet 214.

Although one specific order of different layers in dialysis cartridge 200 is shown in FIG. 3, it should be appreciated that the urease layer 240, zirconium oxide layer 250 and/or carbon layer 260 can be arranged in other suitable orders to optimize the performance of dialysis cartridge 200 according to the objectives of the user. In addition, permeable layers 214, 252 and 262 can be used to separate any of the aforementioned layers in cartridge 110. Permeable layers 214, 252 and 262 can be made from any suitable fluid permeable material such as, for example, filter paper or a hydrophilic membrane. In addition, if desired, any of the urease layer 240, zirconium oxide layer 250, or carbon layer 260 may be provided in a separate cartridge or vessel to permit replacement of one or more layers independently.

During use of dialysis system 200, a pump 278, such as a membrane or peristaltic pump, pumps spent or effluent dialysis fluid through line 280 so as to enter inlets 212 of dialysis cartridge 210. The spent dialysis fluid passes through the different layers of dialysis cartridge 210, so that each layer removes one or more effluent compounds from the spent dialysis fluid stream introduced through line 280. A regenerated dialysis stream exits outlet 214 of dialysis cartridge 210 through regenerated dialysate line 282. One or more suitable dialysis supplements such as calcium, magnesium, potassium, bicarbonate, acetate and/or other suitable electrolytes can be added from one or more sources 270 via one or more substitution pumps 272, which can be any type described for pump 278, to regenerated dialysis line 282 after regenerated dialysis fluid exits dialysis system 200.

Controller 204 controls pumps 272 and 278 as needed to reach the desired dose of additives and to achieve the desired flow through cartridge 210, respectively. Controller 204 can include one or more processors and memories as has been discussed herein to control the other features of dialysis system 200 of FIGS. 4A to 4D. Controller 204 and dialysis system 200 can include any of the alternatives discussed above for controller 4 and dialysis system 2, respectively.

### Methodology

In light of the systems and cartridges discussed herein, a method of managing sodium during a dialysis therapy is provided. The method includes circulating a spent dialysis fluid in a fluid circuit that includes a dialysis system having a dialysis cartridge having a first column with a first cation exchange resin, wherein greater than 90% exchange sites are populated with hydrogen ions and a second column having a second cation exchange resin, wherein greater than 90% of exchange sites are populated with sodium ions. The second column can be parallel with the first column. The method further includes removing ions from the dialysis fluid with the cartridge to produce a regenerated dialysis fluid, and recirculating the regenerated dialysis fluid back to a patient.

Dialysis cartridges 10, 110 and 210 can be used in many different types of dialysis treatment systems including one loop (e.g., peritoneal dialysis) or two loop dialysis (e.g., hemodialysis or peritoneal dialysis) systems. The following discussion of the different components of dialysis systems 2, 100 and 200 applies to any embodiments of the present disclosure. Pursuant to the embodiments of the present disclosure, dialysis systems 2, 100 and 200 can be used to maintain electrolyte concentrations, especially with respect to sodium, and the solution pH of the dialysate at physiologic levels (e.g., 7.3 to 7.5) while removing uremic toxins.

Urea is removed by an enzymatic conversion of urea using urease followed by subsequent removal of the conversion byproducts. In the enzymatic reaction, one mole of urea is decomposed into two moles of ammonia and one mole of carbon dioxide. Ammonia ("NH₃") is primarily (> 95%) present as ammonium ion ("NH₄⁺") because its logarithmic acid dissociation constant ("pKa") of 9.3 is substantially greater than the solution pH. The carbon dioxide that is formed can be present as either dissolved carbon dioxide or as bicarbonate ion, depending on the solution pH. Because the pKa for this equilibrium is 6.1, both species may be present in substantial quantities under conditions of use. In addition, if the solution is in communication with a gas phase, the dissolved carbon dioxide can be in equilibrium with the carbon dioxide present in the gas phase.

In solution, ammonia acts as a base since the formation of ammonium results from the donation of H⁺. Similarly, carbon dioxide ("CO₂") acts as an acid, since the formation of bicarbonate ("HCO₃⁻") donates H+ to solution. The net result of the urease reaction is to increase the pH. In an embodiment, 25 to 250 mg of urease can be used as the urease layer, although other amounts of urease may be used if they are sufficient to convert the urea present in the solution to ammonium and carbon dioxide. Preferably, urease makes up the first layer of dialysis cartridges 10, 110 and 210.

A variety of urease materials can be used. For example, crosslinked enzyme crystals of urease ("Urease-CLEC") can be used. This material is ultra pure and has high specific activity. Therefore, a very small quantity of this urease is sufficient to provide the desired urea-conversions.

The cation exchange resins in any of the embodiments of the present disclosure can be any suitable cation exchange materials (in any suitable form) such as, for example, zirconium phosphate or crosslinked sulfonated polystyrene (e.g., DOWEX® 88 resin). Zirconium phosphate can absorb, under certain conditions, ammonium ion, calcium, magnesium, potassium and sodium. Ammonium ion is removed from solution via an ion exchange process using zirconium phosphate. Zirconium phosphate can contain two counter-ions, hydrogen ("H⁺") and sodium ("Na⁺"). Release of the counter-ions is determined by the solution pH and the current loading state of the resin. In addition to its role as an ion exchange resin, zirconium phosphate also has a considerable buffering capacity. The zirconium phosphate resin possesses excellent capacity for absorbing ammonium, and this capacity is unaffected by changes in equilibrated pH within a given range (pH 6.0-7.2).

The desired pH of the zirconium phosphate will depend, in part, on its location in the resin bed, e.g., the component it is designed to remove. To this end, the zirconium phosphate layer can have a pH of between approximately 2 to about 8. In an embodiment, zirconium phosphate is present in the cartridges in a range of approximately 200 to about 800 grams. The minimum amount of zirconium phosphate necessary is an amount that is sufficient to remove the ammonium that is generated. The level of ammonium generated is determined by the urea that is to be removed by the dialysis cartridges. Thus, the amount of zirconium phosphate required can equal the ammonium to be removed divided by the capacity of the zirconium phosphate to remove ammonium, which can be determined experimentally.

The anion exchange resins in any embodiments of the present disclosure can be any suitable anion exchange materials (in any suitable form) such as, for example, zirconium oxide, or quaternary divinylbenzene polystyrene (e.g., DOWEX® MP725A resin). A convenient choice of the anion exchange resin can be hydrous zirconium oxide in the hydroxide form, noted as "zirconium oxide" in this disclosure.

The zirconium oxide layer can remove phosphates. The zirconium oxide layer, depending on the pH, can also function to remove sodium. In an embodiment, the zirconium oxide layer has a pH of approximately 6 to about 13. The phosphate capacity of the resin is very high; thus, the size of the zirconium oxide layer can be governed by how much phosphate needs to be removed.

The zirconium oxide layer can function to remove any phosphate that may not have been absorbed by the other components of the resin bed. Further, the zirconium oxide layer can be designed to control the pH of the solution leaving the dialysis cartridge. Accordingly, in an embodiment, the zirconium oxide layer, if it is the last layer (not including the carbon layer) of the cartridge, has a pH of approximately 7 to about 9, and in a preferred embodiment, approximately 7.4. Although the zirconium oxide layer can be the last layer (not including the carbon layer), multiple zirconium oxide layers can be used as the last "layer".

Carbon can be used to remove creatinine, uric acid or other organic molecules that still may be present in the dialysis solution. Although the volume of carbon can encompass a wide range, in an embodiment, approximately 50 to about 200 grams of carbon is used in the cartridges. In one preferred embodiment, the carbon will be of the type that has the ability to remove less than 30 grams of glucose from the dialysis solution. Thus, such a carbon layer will not remove an excess amount of glucose from the dialysis solution. Activated carbon sold under the designation LP-50 by Carbochem, Ardmore, Pa., has been found to function satisfactorily in this regard. Other carbons can be used. It should be appreciated that the carbon layer can be located in any order within the dialysis cartridge, although in one preferred embodiment, the carbon layer is the last layer.

In alternative embodiments, the dialysis cartridges can include any number of components layers. It should also be noted that the layers may not have discrete boundaries (e.g., in the form of permeable layers) but may be blended together. For example, it is possible to have a gradient of two materials between the zirconium oxide and the zirconium phosphate layers.

### Therapies

Any of the dialysis systems 2, 100 and 200 discussed herein can be used for peritoneal dialysis ("PD"), hemodialysis ("HD"), hemofiltration ("HF") or hemodiafiltration ("HDF") as shown in FIGS. 4A to 4D, respectively. FIG. 4A illustrates a schematic of a PD treatment being performed on a patient 300. Spent dialysis fluid from patient 300 is recirculated through one of dialysis systems 2, 100 and 200 for treatment/urea removal. Regenerated dialysis is returned to the patient for reuse. The recirculation can be done on a continuous basis ("CFPD"), on a batch basis in which dialysis fluid dwells within patient 300 for a period of time, or on a semi-continuous or tidal basis.

FIG. 4B illustrates a schematic of a hemodialysis ("HD") treatment being performed on patient 300. Blood from patient 300 is pumped through a dialyzer 302, cleaned and returned to patient 300. Spent dialysis fluid from dialyzer 302 is recirculated through one of dialysis systems 2, 100 and 200 for treatment/urea removal. The treated fluid is then returned to dialyzer 302 on a continuous basis to continuously clean the patients' blood. Any of controllers 4, 104 or 204 of systems 2, 100 or 200, respectively, can run any or all portions of the associated HD system.

FIG. 4C illustrates a schematic of a hemofiltration ("HF") treatment technology. HF is a technology similar to HD With hemofiltration, dialysate is not used. Instead, a positive hydrostatic pressure drives water and solutes across the filter membrane of hemofilter 303 from its blood compartment to its filtrate compartment, from which it is drained. The spent dialysis fluid is sent to one of dialysis systems 2, 100 and 200 for treatment/urea removal. The treated fluid is then further purified by being sent through one or more pyrogen filters 304 such as an ultrafilter, pyrogen filter or nanofilter that removes toxins and endotoxins. The resulting replacement fluid is pumped directly into the blood causing a convective cleansing of the patient. As with PD and HD, a net volume of fluid is taken off of the patient as ultrafiltrate to remove excess water that the patient has accumulated between treatments. Any of controllers 4, 104 or 204 of systems 2, 100 or 200, respectively, can run any or all portions of the associated HF system.

FIG. 4D illustrates a schematic of a hemodiafiltration ("HDF") treatment technology. HDF is a combination of HD and HF. Blood is pumped through the blood compartment of dialyzer 302 in a manner similar to HD and HF. Spent dialysate is pulled from dialyzer 302 and cleaned at one of dialysis systems, 2, 100 and 200. The cleaned dialysate is split, some going directly back to dialyzer 302 and some pumped through one or more of a pyrogen filter, nanofilter, or ultrafilter to form a suitable replacement fluid that is pumped directly into the patient's blood line. HDF results in good removal of both large and small molecular weight solutes. Any of controllers 4, 104 or 204 of systems 2, 100 or 200, respectively, can run any or all portions of the associated HDF treatment system.

In alternative embodiments, the present disclosure provides methods including circulating a dialysis fluid in a fluid circuit of a dialysis technology or apparatus incorporating one or more of dialysis systems 2, 100 and 200 in a form that is wearable/portable.

### Examples

By way of example and not limitation, the following examples are illustrative of various embodiments of the present disclosure and further illustrate experimental testing conducted with the dialysis systems in accordance with embodiments of the present disclosure.

### Objectives:

The present experiments demonstrate improved sodium management to maintain sodium level in a therapeutically important range while removing ammonium ions during sorbent dialysis therapy. This is achieved through a parallel column configuration with a first column containing cation exchange resin in acidic form and a second column containing cation exchange resin in sodium. By adjusting the volume flow rate ratio between these two columns, a relatively constant sodium concentration -140 mM can be maintained.

### Experiments:

Two empty ion exchange columns (GE C10/10 columns (product code: 19-5001-01)) were used. The column has an internal diameter ("I.D.") of 1 cm and height of 10 cm. Detailed preparation of columns in either acidic form or sodium form are described in the individual section separately.

The example solution was prepared by mixing ∼1 g ammonium carbonate (207861-1.5 Kg, Sigma-Aldrich) into 2L ACCUSOL® 35 4K⁺ (5B9248, ACCUSOL® dialysis solution for continuous renal replacement therapy, Baxter Healthcare Corporation), containing sodium (140 mEq/L), ammonium (-10 mEq/L), potassium (4 mEq/L), calcium (3.5 mEq/L), magnesium (1 mEq/L), bicarbonate, chloride (113.5 mEq/L) and dextrose (100 mg/dL).

### Example 1: ZrP ion exchange column in acidic form

The ion exchange column in acidic form was prepared by filling an empty chromatographic column (GE C10/10 column: 19-5001-01) with 8.004 g zirconium phosphate resin (from Renal Solutions Inc., Lot B410) and by rinsing with a 500 mL 0.1 M hydrochloride solution at 5 mL/min to ensure the cation exchange column was in acidic form. The column was rinsed with 500 mL deionized ("DI") water at 5 mL/min to ensure that the residual hydrochloride in the column was removed before experimenting.

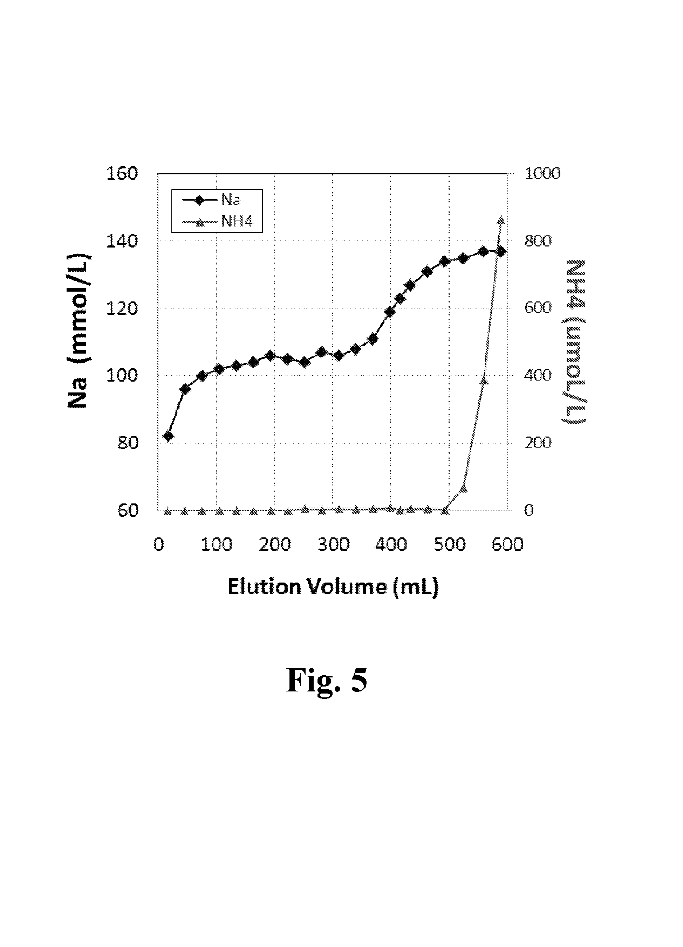

The example solution was used in the experiment and the flow rate was measured at 5.88 mL/min. The samples were collected every five minutes at the outlet of the column, and time zero was defined when the example solution completely replaced the DI water originally in the column. All the samples were analyzed through clinical chemistry methods to measure ion concentration. The results (FIG. 5) indicate that the sodium concentration achieves a plateau of ∼104 mEq/L before sodium breakthrough occurs as the elution volume is between 104 and 310 mL. As the elution continues, ammonium actually replaces sodium until its breakthrough happens. The reduction of sodium is -36 mM.

### Example 2: ZrP ion exchange column in sodium form

The column in sodium form was prepared in a similar fashion by filling an empty column with 3.622 g zirconium phosphate resin followed by 1.984 g active carbon (CR2050C-AW, lot #CA10-2, from Carbon Resources) and by rinsing with 500 mL of saturated sodium bicarbonate solution at 5 mL/min to ensure that this cation exchange column was in sodium form. The column was rinsed with 500 mL DI water at 5 mL/min to ensure that the residual sodium bicarbonate in the column was removed before experimenting.

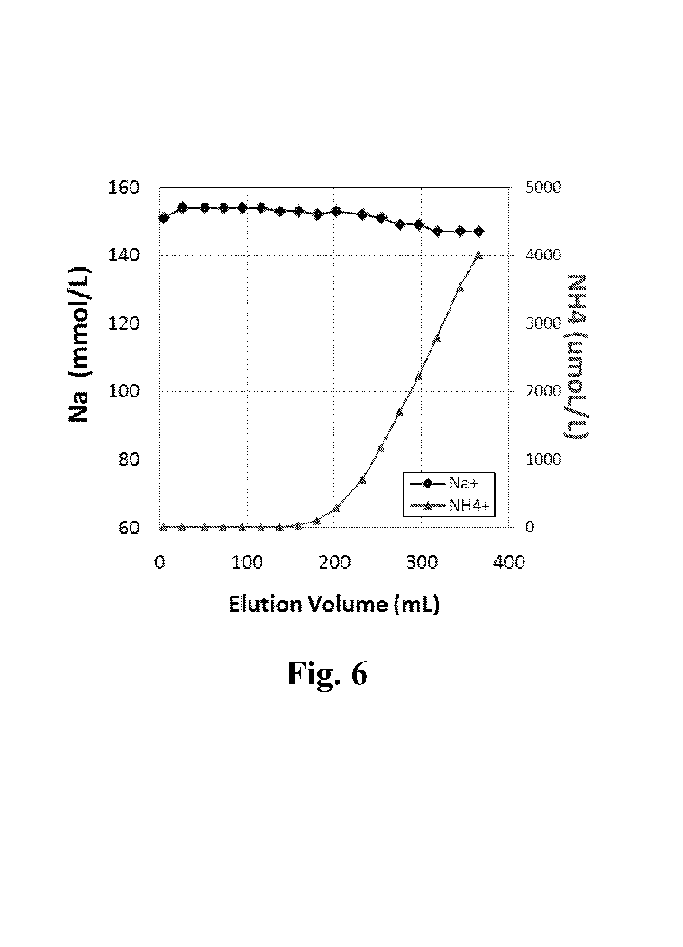

The example solution was used in the experiment and the flow rate was measured to be 4.3 mL/min. The samples were collected every five minutes at the outlet of the column, and time zero was defined when the example solution completely replaced the DI water originally in the column. All the samples were analyzed through clinical chemistry methods to measure ion concentration. The results (FIG. 6) indicate that the sodium concentration increases up to ∼152 mEq/L between 4 and 159 mL. The increase of sodium was ∼12 mM.

### Example 3: Combined ZrP columns in acidic and sodium form

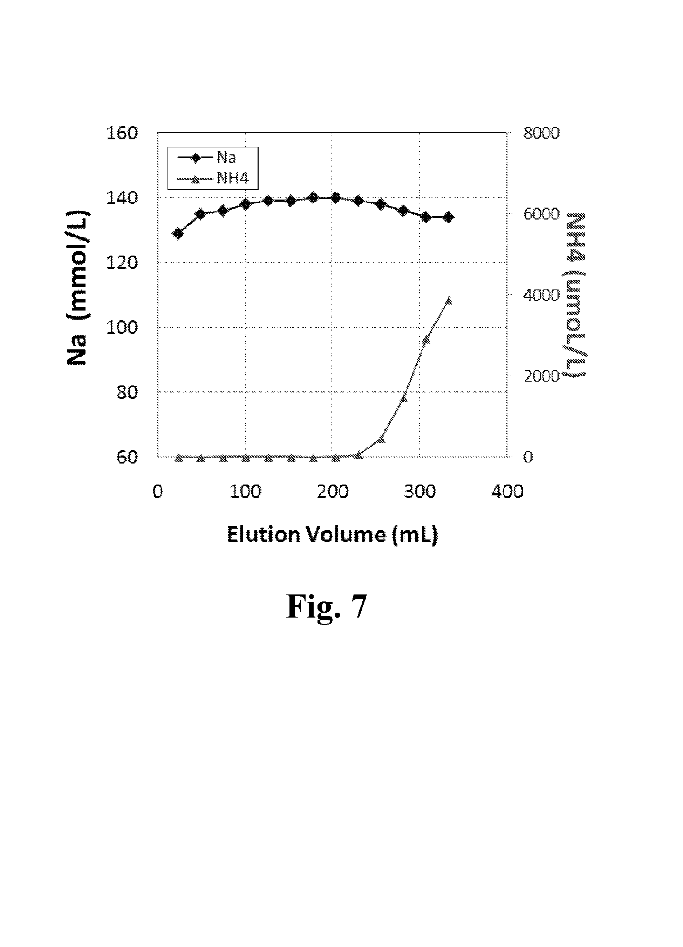

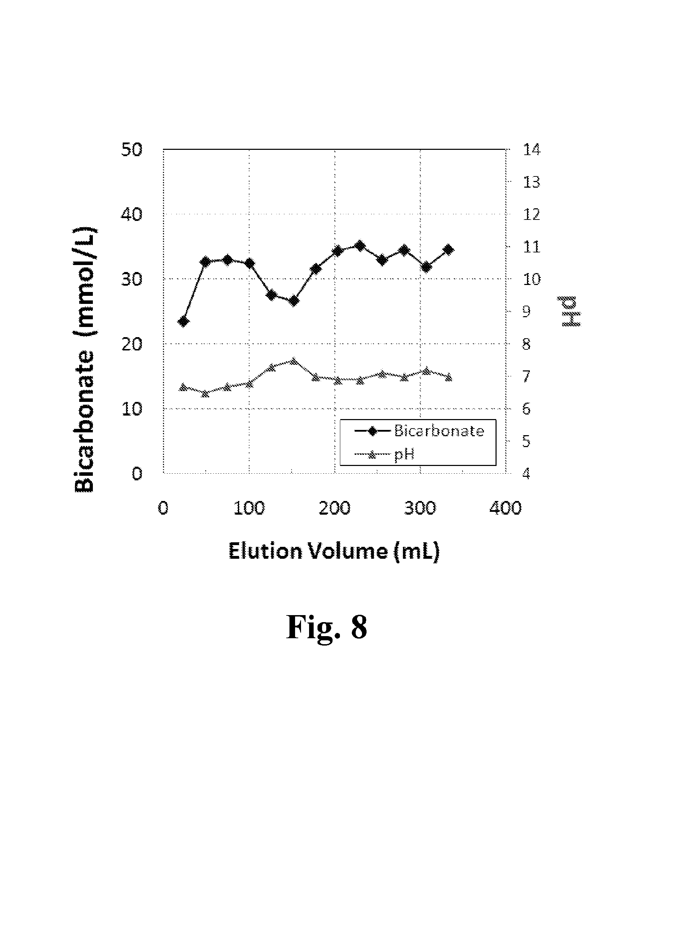

Based on the results from two separated columns, a modification of volume flow rate ratio of 3:1 was made to balance the output sodium level through combined parallel columns. The same columns were used in this experiment. The flow going through this column in acidic form was measured to be 1.61 mL/min, and the flow rate of the column in sodium form was measured to be 4.85 mL/min. The streams out of the two columns were combined into one stream through a Y-shape connector with mixing capability. A sample was collected every four minutes, and time zero was defined when the example solution completely replaced the DI water originally in the columns. All the cations and anions were analyzed through clinical chemistry methods, and the pH was measured. FIG. 7 represents a typical result showing that sodium concentration is maintained relatively constant at ∼140 mM at the elution volume between 101 to 230 mL before ammonium ion breakthrough occurs. FIG. 8 shows that pH is also maintained at a consistent level around 7.

### Conclusions:

These sets of experiments demonstrate improved sorbent dialysis is available by maintaining sodium levels while removing excessive ammonium ion through a parallel cation exchange column configuration in acidic and sodium form. The dialysis systems and methods can be readily implemented in a variety of peritoneal dialysis or hemodialysis therapy including on-site, at-home or portable dialysis systems for improved sodium management.

It should be understood that various changes and modifications to the presently preferred embodiments described herein will be apparent to those skilled in the art. It is therefore intended that such changes and modifications be covered by the appended claims.

## Claims

1. An apparatus (10) for treating spent dialysate comprising first and second fluid flow pathways, the first fluid flow pathway containing a first cation exchange resin (22), **characterized in that**:
the first and second fluid flow pathways are in a parallel arrangement;
greater than 90% of exchange sites of the first cation exchange resin are populated with hydrogen ions,
the second fluid flow pathway contains a second cation exchange resin (32) with greater than 90% of exchange sites of the second cation exchange resin being populated with sodium ions, and
a total ion exchange capacity ratio of the first cation exchange resin (22) compared to the second cation exchange resin (32) ranges from about 1:1 to about 1:5.

2. The apparatus (10) according to claim 1, wherein the apparatus is a dialysate regeneration cartridge.

3. The apparatus (10) according to Claim 1 or 2, wherein greater than 95% of exchange sites of the first cation exchange resin (22) are populated with hydrogen ions and greater than 95% of exchange sites of the second cation exchange resin (32) are populated with sodium ions.

4. The apparatus (10) according to Claim 1 or 2, wherein greater than 99% of exchange sites of the first cation exchange resin (22) are populated with hydrogen ions and greater than 99% of exchange sites of the second cation exchange resin (32) are populated with sodium ions.

5. The apparatus (10) according to any one of the preceding claims, further comprising, in association with the first and second fluid flow pathways, at least one layer of material (50) selected from the group consisting of urease, zirconium oxide, carbon and combinations thereof.

6. The apparatus according to Claim 1, said apparatus (10) further comprising a third fluid flow pathway in substantially parallel flow arrangement with said first and second fluid flow pathways, said third fluid flow pathway comprising an anion exchange resin (222), wherein from about 20% to about 80% of exchange sites of the anion exchange resin (222) are populated with carbonate or bicarbonate ions.

7. The apparatus (10) according to Claim 6, wherein the total ion exchange capacity ratio of the first cation exchange resin (22) compared to the anion exchange resin (222) ranges from about 1:0 to about 1:2.

8. The apparatus (10) according to Claim 6 or 7, wherein greater than 95% of exchange sites of the first cation exchange resin (22) are populated with hydrogen ions, greater than 95% of exchange sites of the second cation exchange resin (32) are populated with sodium ions, and greater than 95% of exchange sites of the anion exchange resin (222) are populated with carbonate or bicarbonate ions.

9. The apparatus (10) according to Claim 6 or 7, wherein greater than 99% of exchange sites of the first cation exchange resin (22) are populated with hydrogen ions, greater than 99% of exchange sites of the second cation exchange resin (32) are populated with sodium ions, from about 40% to about 60% of exchange sites of the anion exchange resin (222) are populated with carbonate or bicarbonate ions, and about 40% to about 60% of exchange sites of the anion exchange resin (222) are populated with hydroxide ions.

10. The apparatus (10) according to any one of Claims 6 to 9, further comprising, in association with the first, second and third fluid flow pathways, at least one layer (50) selected from the group consisting of a urease layer, a zirconium oxide layer, a carbon layer and combinations thereof.

11. The apparatus (10) according to claim 1, wherein the spent dialysate is generated in a dialysis treatment selected from the group consisting of hemodialysis, hemodiafiltration, and peritoneal dialysis.

12. An apparatus (2) for performing a dialysis therapy, comprising a source of dialysate and the dialysate treatment apparatus (10) of Claim 1.

13. The apparatus (1) according to Claim 12, wherein the dialysis therapy is selected from the group consisting of hemodialysis, hemodiafiltration, and peritoneal dialysis.

14. The apparatus (10) according to Claim 2, wherein the cartridge includes:
an inlet (12) and an outlet (14) and defines an interior, wherein the interior includes:
a urease layer (40), and
a zirconium oxide layer (50).

15. The apparatus (10) according to Claim 14, wherein the interior of the cartridge further comprises a carbon layer (60).

16. The apparatus (10) according to Claim 15, wherein the carbon layer (60) is located nearest to the outlet (14).

17. The apparatus (10) according to any one of Claims 14 to 16, wherein the urease layer (40) is positioned closest to the inlet (12).

## Patentansprüche

1. Vorrichtung (10) zum Behandeln von verbrauchtem Dialysat, umfassend einen ersten und einen zweiten Fluidströmungsweg, wobei der erste Fluidströmungsweg ein erstes Kationenaustauschharz (22) enthält, **dadurch gekennzeichnet, dass**:
der erste und der zweite Fluidströmungsweg parallel angeordnet sind;
mehr als 90 % der Austauschstellen des ersten Kationenaustauschharzes mit Wasserstoffionen besiedelt sind,
der zweite Fluidströmungsweg ein zweites Kationenaustauschharz (32) enthält, wobei mehr als 90 % der Austauschstellen des zweiten Kationenaustauschharzes mit Natriumionen besiedelt sind, und
ein Verhältnis der gesamten Ionenaustauschkapazität des ersten Kationenaustauschharzes (22) verglichen mit dem zweiten Kationenaustauschharz (32) von etwa 1:1 bis etwa 1:5 reicht.

2. Vorrichtung (10) nach Anspruch 1, wobei die Vorrichtung eine Dialysatregenerationspatrone ist.

3. Vorrichtung (10) nach Anspruch 1 oder 2, wobei mehr als 95 % der Austauschstellen des ersten Kationenaustauschharzes (22) mit Wasserstoffionen besiedelt sind und mehr als 95 % der Austauschstellen des zweiten Kationenaustauschharzes (32) mit Natriumionen besiedelt sind.

4. Vorrichtung (10) nach Anspruch 1 oder 2, wobei mehr als 99 % der Austauschstellen des ersten Kationenaustauschharzes (22) mit Wasserstoffionen besiedelt sind und mehr als 99 % der Austauschstellen des zweiten Kationenaustauschharzes (32) mit Natriumionen besiedelt sind.

5. Vorrichtung (10) nach einem der vorhergehenden Ansprüche, ferner umfassend, in Verbindung mit dem ersten und dem zweiten Fluidströmungsweg, wenigstens eine Schicht von Material (50), ausgewählt aus der Gruppe bestehend aus Urease, Zirconiumoxid, Kohlenstoff und Kombinationen davon.

6. Vorrichtung nach Anspruch 1, wobei die Vorrichtung (10) ferner einen dritten Fluidströmungsweg umfasst, der im Wesentlichen in paralleler Strömung mit dem ersten und dem zweiten Fluidströmungsweg angeordnet ist, wobei der dritte Fluidströmungsweg ein Anionenaustauschharz (222) umfasst, wobei von etwa 20 % bis etwa 80 % der Austauschstellen des Anionenaustauschharzes (222) mit Carbonat- oder Bicarbonationen besiedelt sind.

7. Vorrichtung (10) nach Anspruch 6, wobei das Verhältnis der gesamten Ionenaustauschkapazität des ersten Kationenaustauschharzes (22) verglichen mit dem Anionenaustauschharz (222) von etwa 1:0 bis etwa 1:2 reicht.

8. Vorrichtung (10) nach Anspruch 6 oder 7, wobei mehr als 95 % der Austauschstellen des ersten Kationenaustauschharzes (22) mit Wasserstoffionen besiedelt sind, mehr als 95 % der Austauschstellen des zweiten Kationenaustauschharzes (32) mit Natriumionen besiedelt sind und mehr als 95 % der Austauschstellen des Anionenaustauschharzes (222) mit Carbonat- oder Bicarbonationen besiedelt sind.

9. Vorrichtung (10) nach Anspruch 6 oder 7, wobei mehr als 99 % der Austauschstellen des ersten Kationenaustauschharzes (22) mit Wasserstoffionen besiedelt sind, mehr als 99 % der Austauschstellen des zweiten Kationenaustauschharzes (32) mit Natriumionen besiedelt sind, von etwa 40 % bis etwa 60 % der Austauschstellen des Anionenaustauschharzes (222) mit Carbonat- oder Bicarbonationen besiedelt sind und von etwa 40 % bis etwa 60 % der Austauschstellen des Anionenaustauschharzes (222) mit Hydroxidionen besiedelt sind.

10. Vorrichtung (10) nach einem der Ansprüche 6 bis 9, ferner umfassend, in Verbindung mit dem ersten, dem zweiten und dem dritten Fluidströmungsweg, wenigstens eine Schicht (50), ausgewählt aus der Gruppe bestehend aus einer Ureaseschicht, einer Zirconiumoxidschicht, einer Kohlenstoffschicht und Kombinationen davon.

11. Vorrichtung (10) nach Anspruch 1, wobei das verbrauchte Dialysat in einer Dialysebehandlung erzeugt wird, ausgewählt aus der Gruppe bestehend aus Hämodialyse, Hämodiafiltration und Peritonealdialyse.

12. Vorrichtung (2) zum Durchführen einer Dialysetherapie, umfassend eine Dialysatquelle und die Dialysatbehandlungsvorrichtung (10) nach Anspruch 1.

13. Vorrichtung (1) nach Anspruch 12, wobei die Dialysetherapie ausgewählt ist aus der Gruppe bestehend aus Hämodialyse, Hämodiafiltration und Peritonealdialyse.

14. Vorrichtung (10) nach Anspruch 2, wobei die Patrone Folgendes beinhaltet:
einen Einlass (12) und einen Auslass (14) sowie einen Innenraum definiert, wobei der Innenraum Folgendes beinhaltet:
eine Ureaseschicht (40) und
eine Zirconiumoxidschicht (50).

15. Vorrichtung (10) nach Anspruch 14, wobei der Innenraum der Patrone ferner eine Kohlenstoffschicht (60) umfasst.

16. Vorrichtung (10) nach Anspruch 15, wobei sich die Kohlenstoffschicht (60) am nächsten zum Auslass (14) befindet.

17. Vorrichtung (10) nach einem der Ansprüche 14 bis 16, wobei sich die Ureaseschicht (40) am nächsten zum Einlass (12) befindet.

## Revendications

1. Appareil (10) destiné à traiter du dialysat usagé comprenant des première et deuxième voies d'écoulement de fluide, la première voie d'écoulement de fluide contenant une première résine échangeuse de cations (22), **caractérisé en ce que** :
les première et deuxième voies d'écoulement de fluide sont dans un agencement parallèle ;
plus de 90 % des sites d'échange de la résine échangeuse de cations sont peuplés d'ions hydrogène,
la deuxième voie d'écoulement de fluide contient une deuxième résine échangeuse de cations (32), plus de 90 % des sites d'échange de la deuxième résine échangeuse de cations étant peuplés d'ions sodium, et
un rapport de capacité d'échange d'ions total entre la première résine échangeuse de cations (22) et la deuxième résine échangeuse de cations (32) va d'environ 1:1 à environ 1:5.

2. Appareil (10) selon la revendication 1, dans lequel l'appareil est une cartouche de régénération de dialysat.

3. Appareil (10) selon la revendication 1 ou 2, dans lequel plus de 95 % des sites d'échange de la première résine échangeuse de cations (22) sont peuplés d'ions hydrogène et plus de 95 % des sites d'échange de la deuxième résine échangeuse de cations (32) sont peuplés d'ions sodium.

4. Appareil (10) selon la revendication 1 ou 2, dans lequel plus de 99 % des sites d'échange de la première résine échangeuse de cations (22) sont peuplés d'ions hydrogène et plus de 99 % des sites d'échange de la deuxième résine échangeuse de cations (32) sont peuplés d'ions sodium.

5. Appareil (10) selon l'une quelconque des revendications précédentes, comprenant en outre, en association avec les première et deuxième voies d'écoulement de fluide, au moins une couche de matériau (50), choisie dans le groupe consistant en l'uréase, l'oxyde de zirconium, le carbone et des combinaisons de ceux-ci.

6. Appareil selon la revendication 1, ledit appareil (10) comprenant en outre une troisième voie d'écoulement de fluide dans un agencement d'écoulement sensiblement parallèle avec lesdites première et deuxième voies d'écoulement de fluide, ladite troisième voie d'écoulement de fluide comprenant une résine échangeuse d'anions (222), dans lequel d'environ 20 % à environ 80 % des sites d'échange de la résine échangeuse d'anions (222) sont peuplés d'ions carbonate ou bicarbonate.

7. Appareil (10) selon la revendication 6, dans lequel le rapport de capacité d'échange d'ions total de la première résine échangeuse de cations (22) comparée à la résine échangeuse d'anions (222) va d'environ 1:0 à environ 1:2.

8. Appareil (10) selon la revendication 6 ou 7, dans lequel plus de 95 % des sites d'échange de la première résine échangeuse de cations (22) sont peuplés d'ions hydrogène, plus de 95 % des sites d'échange de la seconde résine échangeuse de cations (32) sont peuplés d'ions sodium, et plus de 95 % des sites d'échange de la résine échangeuse d'anions (222) sont peuplés d'ions carbonate ou bicarbonate.

9. Appareil (10) selon la revendication 6 ou 7, dans lequel plus de 99 % des sites d'échange de la première résine échangeuse de cations (22) sont peuplés d'ions hydrogène, plus de 99 % des sites d'échange de la seconde résine échangeuse de cations (32) sont peuplés d'ions sodium, d'environ 40 % à environ 60 % des sites d'échange de la résine échangeuse d'anions (222) sont peuplés d'ions carbonate ou bicarbonate et d'environ 40 % à environ 60 % des sites d'échange de la résine d'anions (222) sont peuplés d'ions hydroxyde.

10. Appareil (10) selon l'une quelconque des revendications 6 à 9, comprenant en outre, en association aux première, deuxième et troisième voies d'écoulement de fluide, au moins une couche (50) choisie dans le groupe consistant en une couche d'uréase, une couche d'oxyde de zirconium, une couche de carbone et des combinaisons de celles-ci.

11. Appareil (10) selon la revendication 1, dans lequel le dialysat usagé est généré dans un traitement de dialyse choisi dans le groupe consistant en l'hémodialyse, l'hémodiafiltration et la dialyse péritonéale.

12. Appareil (2) permettant de réaliser une thérapie de dialyse, comprenant une source de dialysat et l'appareil de traitement de dialysat (10) selon la revendication 1.

13. Appareil (1) selon la revendication 12, dans lequel la thérapie de dialyse est choisie dans le groupe consistant en l'hémodialyse, l'hémodiafiltration et la dialyse péritonéale.

14. Appareil (10) selon la revendication 2, dans lequel la cartouche comprend :
une admission (12) et un refoulement (14) et définit un intérieur, dans lequel l'intérieur comprend :
une couche d'uréase (40), et
une couche d'oxyde de zirconium (50).

15. Appareil (10) selon la revendication 14, dans lequel l'intérieur de la cartouche comprend en outre une couche de carbone (60).

16. Appareil (10) selon la revendication 15, dans lequel la couche de carbone (60) est située la plus proche du refoulement (14).

17. Appareil (10) selon l'une quelconque des revendications 14 à 16, dans lequel la couche d'uréase (40) est positionnée la plus près de l'admission (12).
